(19) Europäisches Patentamt
European Patent Office
Office européen des brevets

(11) EP 4 550 351 A1

(12) **EUROPEAN PATENT APPLICATION**

(43) Date of publication:
07.05.2025 Bulletin 2025/19

(21) Application number: 23216186.9

(22) Date of filing: 13.12.2023

(51) International Patent Classification (IPC):
*G16H 50/20* (2018.01)    *A61B 5/00* (2006.01)
*G16H 50/30* (2018.01)    *G16H 50/50* (2018.01)
*G16H 50/70* (2018.01)

(52) Cooperative Patent Classification (CPC):
**G16H 50/70; A61B 5/412; A61B 5/7264; A61B 5/7275; G16H 50/20; G16H 50/30; G16H 50/50;** G16H 10/60

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: 01.11.2023 PCT/CN2023/128992

(71) Applicant: **Koninklijke Philips N.V.**
**5656 AG Eindhoven (NL)**

(72) Inventors:
• YUAN, yana
Eindhoven (NL)
• TIAN, Cong
Eindhoven (NL)
• JIANG, Zeyu
5656AG Eindhoven (NL)
• FU, Yong
Eindhoven (NL)

(74) Representative: **Philips Intellectual Property & Standards**
**High Tech Campus 52**
**5656 AG Eindhoven (NL)**

(54) **SEPSIS DETECTION**

(57) A mechanism for predicting a probability of sepsis in a subject. The mechanism includes obtaining and standardizing at least one of each of a vital sign value, a demographic value, and a symptom status value. These standardized values are then input into a machine-learning model trained to predict, based on the input values, a probability of the subject having sepsis.

100

- Obtaining a plurality of input values — 110
- Generating standardized input values — 120
- Providing the standardized input values to a machine-learning model — 130

**FIG. 1**

EP 4 550 351 A1

**Description**

FIELD OF THE INVENTION

This invention relates to the field of sepsis detection, and in particular, to the field of predicting a probability of sepsis.

BACKGROUND OF THE INVENTION

Sepsis is a syndromic response to infection and is frequently a final common pathway to death from many infectious diseases worldwide. The global burden of sepsis is difficult to ascertain, although a recent scientific publication estimated that it is still a leading cause of death worldwide, accounting for 11 million sepsis-related deaths annually, representing 9.7% of all global death.
Sepsis can be the clinical manifestation of infections acquired in community and healthcare facilities. Healthcare-associated infections are one of, if not the, most frequent type of adverse event to occur during care delivery and affect hundreds of millions of patients worldwide every year. Since these infections are often resistant to antibiotics, they can rapidly lead to deteriorating clinical conditions.
Antimicrobial resistance is a major factor in clinical unresponsiveness to treatment and rapid evolution to sepsis and septic shock. Sepsis patients with resistant pathogens have been found to have a higher risk of hospital mortality. Early diagnosis and timely and appropriate clinical management of sepsis is therefore crucial to increase the likelihood of patients' survival.
There are well-known and frequently used clinical rules for medical patients in emergency departments (ED), including the quick Sequential Organ Failure Assessment (qSOFA) score, the Systemic Inflammatory Response Syndrome criteria (SIRS), and the Modified Early Warning Score (MEWS). Sepsis can be challenging to quickly identify, however, due to its non-specific symptoms.
Several factors contribute to the delayed recognition of sepsis, including lack of awareness among healthcare providers, failure to recognize the severity of the patient's condition, and delays in obtaining laboratory tests or starting treatment. Additionally, comorbidities such as diabetes or chronic kidney disease can complicate the diagnosis of sepsis and make it more difficult to recognize.
The above-mentioned clinical rules have been the results of efforts to enhance early sepsis recognition, however, these systems have limitations including not detecting all cases of sepsis and falsely identifying individuals who do not have sepsis.

SUMMARY OF THE INVENTION

The invention is defined by the claims.
According to examples in accordance with an aspect of the invention, there is provided a computer-implemented method for predicting a probability of sepsis in a subject.
The computer-implemented method comprises: obtaining a plurality of input values comprising: at least one vital sign value of the subject; at least one demographic value of the subject; at least one symptom status value of the subject; generating standardized input values by standardizing each input value to a value within a respective range or one of a set of predetermined values; and providing the standardized input values as input to a machine-learning model, the machine-learning model being trained to predict, from the standardized input values, a sepsis risk score indicating a probability of the subject having sepsis.
Proposed concepts thus aim to provide schemes, solutions, concepts, designs, methods and systems pertaining to predicting a probability of sepsis in a subject. In particular, embodiments aim to provide a method for predicting a probability of sepsis in a subject by obtaining and standardizing at least one of each of a vital sign value, a demographic value, and a symptom status value. These standardized values can then be input into a machine-learning model trained to predict, based on the input values, a probability of the subject having sepsis. For example, training of said machine-learning model may comprise five-fold cross-validation, feature importance ranking and/or a loss function.
In other words, it is proposed that by obtaining and utilizing vital sign values, demographic values, and symptom status values, a machine-learning model can be used to predict a probability of a subject having sepsis. In this way, a probability of the subject having sepsis can be provided in a timely and effective manner, i.e., without having to wait for laboratory-based predictors, which can be time-consuming to obtain. A clinician is also not required to manually review all of the subject's data in order to come to a conclusion regarding the subject's risk of sepsis, but rather can simply confirm the diagnosis of each patient after reviewing the predicted probability of sepsis.
Further, by standardizing the vital sign, demographic, and symptom status values, the use of a machine-learning model is made more effective and/or efficient. For instance, whether or not a symptom is present in a subject can be standardized to simply a 1 or 0 so that the machine-learning model can more effectively and/or efficiently process this information. Likewise, quantitative features such as heart rate or age can be standardized, i.e., between 0 and 1 or between 0 and 10, such that the machine-learning model can more effectively and/or efficiently utilize all of the input data to provide a more reliable sepsis risk score.

This invention may be of particular use in clinical settings where vital sign values of a subject are already being measured or can easily be obtained. Likewise, demographic values and symptom status values can easily be obtained in a clinical setting, and thus a sepsis risk score for the subject can be efficiently (and potentially continuously) provided, while the subject is staying in the clinical setting. In other words, information which is typically already obtained in clinical settings can be leveraged in order to efficiently and/or effectively provide a probability of the subject having sepsis.

In this way, it is not necessary to wait for laboratory results in order to give an indication of whether the subject has sepsis or not. Furthermore, the use of a machine-learning model to process the obtained information and provide a sepsis risk score frees up important clinician time for other matters which require their attention.

Ultimately, an improved method for predicting a probability of sepsis in a subject may be provided.

In some embodiments, if an input value comprises a categorical value, standardizing the input value may comprise setting the categorical value to one of a set of predetermined values; and/or if an input value comprises a numerical value, standardizing the input value may comprise standardizing the numerical value to a value within the respective range of predetermined values. This provides an effective and/or efficient method of standardizing the different types of values which may be input. For example, a triage level of a subject is a categorical value comprising levels I, II, III, and IV and so may be set to one of a set of predetermined values comprising 1, 2, 3, and 4 respectively. In another example, a subject's body temperature is a numerical value and so may be standardized to a respective range such as between 0 and 1 or 0 and 10.

In some embodiments, each at least one symptom status value may be associated with a different symptom, and comprises a value indicating the presence or absence of the associated symptom. For example, after standardization, if the subject has a symptom associated with the symptom status value, the standardized input value can be set to 1, and if the subject does not, the standardized input value can be set to 0. In this way, the symptom status value(s) can be standardized to the same (or a similar) range as, for example, the vital sign values and/or the demographic values, while still providing a binary indication. For instance, the symptom status value can indicate whether the subject has a fever or not, whether they have a stomach ache or not, whether they have altered consciousness or not, etc.

In some embodiments, the machine-learning model may comprise at least one of: a neural network; a decision tree; a linear model; and/or a logistic regression model. These are all types of machine-learning models which have been found to be useful for the present purpose.

In some embodiments, the computer-implemented method may further comprise comparing the sepsis risk score to a predetermined threshold score, and determining a sepsis risk value responsive to the comparison. In this way, the sepsis risk score can be more easily interpreted. For instance, the sepsis risk score can be compared to a predetermined threshold score, and if the risk score is above the threshold, it can be determined that the subject is at risk of (having) sepsis. Conversely, if the risk score is below the threshold, it can be determined that the subject is not at risk of sepsis. In other words, the sepsis risk value may comprise two possible values: that the subject is at risk of sepsis or that the subject is not at risk of sepsis.

In some embodiments, the predetermined threshold score may be configurable by a user. In other words, a user, such as a clinician, can adjust the threshold score to their preference. For instance, a user may find that if the threshold is set at 50%, too many subjects are being classified as being at risk of sepsis when, in reality, they are not. The user can then adjust the threshold to 70%, for example, so that only those who are determined to have a higher probability of having sepsis are labelled as being at risk of sepsis.

In some embodiments, the computer-implemented method may further comprise generating a first display control signal, the first display control signal indicating the sepsis risk value. For example, this allows a clinician to easily see whether the subject is at risk of sepsis or not.

In some embodiments, the at least one vital sign value may comprise at least one of: heart rate; body temperature; SpO2; systolic blood pressure; diastolic blood pressure; mean arterial pressure; and/or respiration rate. These are vital sign values which are useful for helping to determine a probability of a subject having sepsis, while also typically being readily available in a clinical setting.

In some embodiments, if at least two of the input values are known to be highly correlated, then only one of the said input values may be standardized and subsequently processed by the machine-learning model. If the input values are highly correlated, the input of more than one of them does not provide any more useful information while increasing the processing required. Discarding all but one of the values thus makes the method more efficient while maintaining its effectiveness. For example, if at least two of: systolic blood pressure; diastolic blood pressure; and mean arterial pressure are obtained, then only one of the said values may be standardized and subsequently processed by the machine-learning model as these vital sign values are typically highly correlated.

In some embodiments, the computer-implemented method may further comprise discarding an input value if the input value is not obtained with a reliability higher than a predetermined threshold. This increases the efficiency of the method by not processing inputs which are unreliable and so unlikely to provide useful information. In other words, this feature can be understood as if the input value cannot be obtained with a high reliability, then the input value can be discarded and not subsequently processed.

In some embodiments, the at least one demographic value may comprise at least one of: age; sex; race; mode of arrival; and/or triage level. These are useful values for helping to determine the risk of the subject having sepsis. Mode of arrival can be understood as whether the subject arrived by ambulance or walked-in, etc. A triage level is a classification or prioritization assigned to patients, incidents, or tasks based on their severity or urgency to determine the order of treatment or attention.

In some embodiments, the at least one symptom status value may comprise at least one value indicating the presence or absence of one of the following: acutely altered mental status; abnormally frequent micturition; abnormally high body temperature; stomach ache; fever; inappetence; altered consciousness; and/or rigors. These are all symptoms which can arise due to sepsis.

In some embodiments, the computer-implemented method may further comprise generating a second display control signal, the second display control signal indicating the sepsis risk score. For example, this allows a clinician to easily see the probability of the subject having sepsis.

A computer program product comprising computer program code means which, when executed on a computing device having a processing system, cause the processing system to perform all of the steps of the computer-implemented method according to any herein disclosed method.

According to another aspect of the invention, there is provided a processing system for predicting a probability of sepsis in a subject, the processing system comprising: an input unit configured to obtain a plurality of input values comprising: at least one vital sign value of the subject; at least one demographic value of the subject; and at least one symptom status value of the subject; and a processing unit configured to: generate standardized input values by standardizing each input value to a value a respective range or one of a set of predetermined values; and provide the standardized input values as input to a machine-learning model, the machine-learning model being trained to predict, from the standardized input data, a sepsis risk score indicating a probability of the subject having sepsis.

Thus, there may be proposed concepts for predicting a probability of sepsis in a subject, and this may be done based on the processing input vital sign, demographic, and symptom status values.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 is a simplified flow diagram of a computer-implemented method for predicting a probability of sepsis in a subject according to a proposed embodiment;
Fig. 2 is a flow diagram of a training method for a machine-learning model for predicting a probability of sepsis in a subject according to a proposed embodiment;
Fig. 3 is a more in-depth flow diagram of a computer-implemented method for predicting a probability of sepsis in a subject according to a proposed embodiment;
Fig. 4 is a simplified block diagram of a processing system for predicting a probability of sepsis in a subject according to a proposed embodiment; and
Fig. 5 illustrates an example of a computer within which one or more parts of an embodiment may be employed.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a computer-implemented method for predicting a probability of sepsis in a subject. This can be achieved by obtaining and standardizing at least one of each of a vital sign value, a demographic value, and a symptom status value. These standardized values can then be input into a machine-learning model trained to predict, based on the input values, a probability of the subject having sepsis. For example, training of said machine-learning model may comprise five-fold cross-validation, feature importance ranking and/or a loss function.

In other words, it is proposed that by obtaining and utilizing vital sign values, demographic values, and symptom status values, a machine-learning model can be used to predict a probability of a subject having sepsis. In this way, a probability of the subject having sepsis can be provided in a timely and effective manner, i.e., without having to wait for laboratory-based predictors, which can be time-consuming to obtain. A clinician is also not required to manually review all of the subject's data in order to come to a conclusion regarding the subject's risk of sepsis, but rather can simply confirm the diagnosis of each patient after reviewing the predicted probability of sepsis.

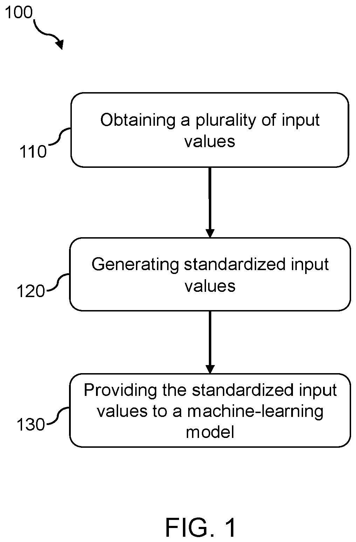

Referring now to Fig. 1, there is depicted a simplified flow diagram of a computer-implemented method 100 for predicting a probability of sepsis in a subject.

The method 100 begins with step 110 of obtaining a plurality of input values. The input values comprise: at least one vital sign value of the subject; at least one demographic value of the subject; and at least one symptom status value of the subject.

In this embodiment, the at least one vital sign value comprises at least one of: heart rate; body temperature; SpO2; systolic blood pressure; diastolic blood pressure; mean arterial pressure; and/or respiration rate. These are vital sign values which are useful for helping to determine a probability of a subject having sepsis, while also typically being readily available in a clinical setting. However, it will be appreciated that any vital sign value which could be informative of the presence of sepsis can be used as an input value.

Vital sign values can also be referred to as time-series information, in that they are expected to change over time.

In this embodiment, the at least one demographic value comprises at least one of: age; sex; race; mode of arrival; and/or triage level. These are useful values for helping to determine the risk of the subject having sepsis. Mode of arrival can be understood as whether the subject arrived by ambulance or walked-in, etc. A triage level is a classification or prioritization assigned to patients, incidents, or tasks based on their severity or urgency to determine the order of treatment or attention. However, it will be appreciated that any demographic value which could be informative of the presence of sepsis can be used.

In this embodiment, the at least one symptom status value comprises at least one value indicating the presence or absence of one of the following: acutely altered mental status; abnormally frequent micturition; abnormally high body temperature; stomach ache; fever; inappetence; altered consciousness; and/or rigors. These are all symptoms which can arise due to sepsis. Acutely altered mental status refers to a sudden and significant change in a person's cognitive or emotional functioning, often indicative of a medical or neurological emergency. Abnormally frequent micturition refers to the condition in which a person experiences an unusually high frequency of urination. Abnormally high body temperature, or hyperthermia, is a medical condition where a person's core body temperature exceeds the normal range of around 97.8°F (36.5°C) to 99.1°F (37.3°C). Altered consciousness refers to a change in an individual's state of awareness, cognition, or responsiveness. Rigors, also known as chills, are a sudden and intense sensation of cold and shivering typically accompanied by fever, often associated with an infection or illness. However, it will be appreciated that any symptom status values which could be informative of the presence of sepsis can be used. These symptom status values can be derived, for example, from an Electronic Medical Record (EMR) system, in which clinicians will record the symptom status values when patient visit the hospital.

Step 120 comprises generating standardized input values by standardizing each input value to a value within a respective range or one of a set of predetermined values. Standardizing each input value to a value within a respective range or one of a set of predetermined values refers to the process of transforming data so that each value can be placed on a consistent and uniform scale (e.g., dependent on the type of value). This is done to make it easier to compare and analyze different variables in a dataset (for instance, by a machine-learning model). Standardization helps ensure fair and meaningful comparisons between different variables and to help algorithms perform better at least by making their calculations more numerically stable.

In one or more embodiments, if an input value comprises a categorical value, standardizing the input value comprises setting the value to one of a set of predetermined values. For example, a triage level of a subject is a categorical value comprising levels I, II, III, and IV and so may be set to one of a set of predetermined values comprising 1, 2, 3, and 4 respectively.

In one or more embodiments, if an input value comprises a numerical value, standardizing the input value comprises standardizing the value to a respective range of predetermined values. For instance, a subject's body temperature is a numerical value and so may be standardized to a respective range such as between 0 and 1 or 0 and 10. As another example, a subject's sex is a binary value and so may be set to either 0 or 1 for male or female respectively.

These approaches provide and effective and/or efficient mechanisms of standardizing different types of values for the input value(s).

In some embodiments, each of the at least one symptom status value is associated with a different symptom, and comprises a value indicating the presence or absence of the associated symptom. For example, if the subject has a symptom associated with the symptom status value, the standardized symptom status value can be set to 1, and if the subject does not, the standardized symptom status value can be set to 0. In this way, the symptom status value(s) can be standardized to the same range as, for example, one or more of the vital sign values and/or the demographic values, while still providing a binary indication. In other embodiments, however, any suitable method of standardization may be performed.

Step 130 comprises providing the standardized input values as input to a machine-learning model. The machine-learning model is trained to predict, from the standardized input values, a sepsis risk score indicating a probability of the subject having sepsis. For clarity, providing the standardized input values as input to the machine-learning model comprises the model actually processing the provided inputs to ultimately output the sepsis risk score. For example, the standardized input values can be provided to the machine-learning model as a matrix or a table, wherein a row represents a single subject and a column represents a single standardized input value.

In this embodiment, the machine-learning model comprises at least one of: a neural network; a decision tree; a linear model; and/or a logistic regression model. These are all types of machine-learning models which have been found to be useful for the present purpose. However, it will be appreciated that any other suitable form of machine-learning model may be used.

A machine-learning algorithm is any self-training algorithm that processes input data in order to produce or predict output data. Here, the input data comprises the standardized input values (e.g., standardized vital sign values, demographic values, and symptom status values of the subject) and the output data comprises a sepsis risk score indicating a probability of the subject having sepsis.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives. Particularly, in one embodiment, an ensemble of machine learning models is trained and used for better predictions, not only relying on one single model.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g., the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

A decision tree algorithm processes input data through a tree of nodes. In the tree of nodes, each successive node splits into two or more further nodes until reaching a terminal or end node. When performing the decision tree algorithm using the tree of nodes, at each node, a decision is made as to which further node to move to next based on the input data. The end node defines the outcome of the decision tree algorithm, and therefore the machine-learning algorithm.

Current efforts to enhance early sepsis recognition in emergency departments (ED) include using clinical decision support tools and sepsis protocols, such as the SIRS, the qSOFA, and the MEWS. However, these scoring systems have limitations such as not detecting all cases of sepsis and falsely identifying individuals who do not have sepsis. It is also commonly impossible to complete all the needed sepsis screening workflow steps during an urgent period in the ED. This is because it can be difficult to get a SOFA score and lab test results in a short time window.

Machine-learning models (i.e., AI models) have performed a role in prediction or classification tasks in many clinical studies. It has been realized that it would be efficient to support clinicians in detecting sepsis or septic shock by integrating AI models trained on clinical datasets into the clinical workflows. Limitations in current AI sepsis prediction research studies include lack of standardization in defining sepsis stages, small sample sizes, absence of comparison with existing scoring systems, and lack of clarity on integration into clinical workflows and patient outcomes. Further, many sepsis prediction models rely on lab-based predictors which are often time-consuming to obtain.

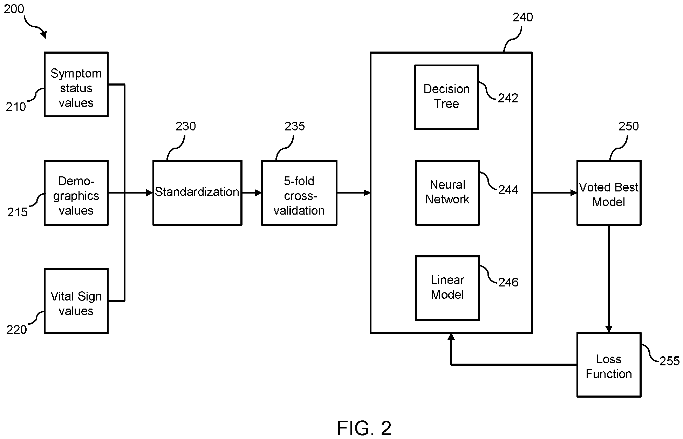

To solve many of these issues, elements proposed in the present disclosure are conceptually divisible into one or more parts. A first part comprises training a machine-learning model with multimodal deep learning methods (e.g., as shown in Fig. 2). A second part comprises configuring the machine-learning model to be able to predict sepsis using minimal EMR and informatics platform variables (including personal/demographic information, time-series/vital sign information, and symptoms information) without laboratory test results. A third part includes configuring a warning score or threshold of the system to be flexibly adjustable to optimize the prediction performance to meet clinical requirements, such that the threshold can be increased to decrease false positive rates.

Referring now to Fig. 2, there is depicted a flow diagram of a training method 200 for a machine-learning model for predicting a probability of sepsis in a subject according to a proposed embodiment.

The training method 200 starts with the obtaining of training data, i.e., symptom status values 210, demographic values 215, and vital sign values 220. In step 230, the training data is standardized. For example, in an embodiment of the proposed method, symptoms and physical characteristics from a structured EMR are extracted. Fixed features are then identified and features with a high-missing rate are dropped. For instance, if one feature cannot be obtained with a high enough rate (e.g., if 20/30% of the feature cannot be obtained) then the feature is discarded. This means that this input value will not be used for training the machine-learning model. For example, missing data can be defined as data that was not recorded during a patient's visit to the ED, while out-of-range values can be defined as those beyond the upper and lower limits of physiological ranges. In short, missing data/features means that there is no record of that data in the system, or that the feature is not in the normal range, and thus not reliable and so dropped/discarded.

For example, at this stage, all input data can be merged into one table based on the same patient ID, and the numerical input data can then be standardized as 0-1 vectors in the table before being input into the model to-be-trained. For the categorical value input data, if one input data value has two categories, it is standardized as 0 or 1, while for triage level, as it has four categories, it is standardized as 1, 2, 3, or 4 respectively. As different features are obtained from different data sources, such as demographic and symptoms information from an EMR system and time series information from another system (e.g., an IntelliSpace Critical Care and Anesthesia (ICCA) system), feature aggregation can help the model to read the raw data from one single table and therefore more effectively and/or efficiently perform data preprocessing (for example, data checking for errors and data standardization). It should be noted that standardizing to 0-1 vectors is merely presented as an example. The ultimate aim for standardization is to put all features (i.e., input values) into one uniform dimension.

The below is provided as an example of a table in which input values (features) for multiple patients have been aggregated and standardized:

|  | Age | HR | Spo2 | Sex | Mode of arrival | Symptom | Triage |
|---|---|---|---|---|---|---|---|
| Patient 1 | 0.5 | 0.3 | 0.6 | 1 | 1 | 1 | 1 |
| Patient 2 | 0.2 | 0.7 | 0.9 | 0 | 0 | 0 | 2 |
| .... |  |  |  |  |  |  |  |
| .... |  |  |  |  |  |  |  |
| Patient m | 1 | 0.8 | 0.4 | 1 | 1 | 1 | 3 |

For example, the above table can be understood as indicating that patient 1 is of a median age (such as, for example, 50 years old), has a relatively low heart rate, has a slightly above average blood oxygen reading, is male, arrived by ambulance, has a particular symptom, and has a triage level of I.

In step 235, the standardized training data undergoes five-fold cross-validation. Five-fold cross-validation is a technique used in machine learning and statistical modeling, where a dataset is divided into five equal subsets. It involves training a model on two of these five subsets, using two of the remaining three subsets for validation, and using one of the remaining three subsets for testing, and this process is repeated five times with each subset serving as the test set exactly once. After each iteration, a model will be obtained. The five models from the five iterations are then compared with each other to assess the model's performance and generalization ability. This approach helps evaluate how well a machine learning model can perform on unseen data and provides a more robust estimate of its performance compared to a single train-test split.

After being split into five equal subsets, training data (i.e., two subsets) is provided to several different types of machine-learning models 240, such as, a decision tree 242, a neural network 244, a linear model 246, and etc. Thus, for one machine-learning model, five model candidates will be obtained based on the abovementioned five-fold cross-validation. The performance of those five model candidates is compared with each other to find the best model candidate therefrom. In other words, best model candidates are obtained for each type of machine-learning model by performing training of each machine-learning model. The best model candidate for each type of machine-learning model is then compared to one another to find the voted best model in step 250.

A loss function 255 is then used to iteratively improve the voted best model 250, providing information back to the several different types of machine-learning models 240.

In more detail, data imbalance or imbalanced classes can be a common problem in classification tasks. Therefore, in the present training method 200, the sepsis class distribution is oversampled by using the SMOTE (Synthetic Minority Oversampling Technique) method. In this way, a random sample is picked from the minority class first, and then its neighboring samples can be found and added between the chosen sample and its neighbors. For example, in one original sample set of 10,000 people, 9,900 people do not have sepsis, while 100 do. With SMOTE, the sample is modified so that 9,900 people now have sepsis, while still maintaining the 9,900 people who do not have sepsis. In this way, the dataset is made more balanced while growing the dataset to (9,900 * 2=) 19,800 people.

During training, the dataset may be divided into five portions by patient, for example, portion 1, portion 2, portion 3, portion 4, and portion 5. One of the five portions will be used as a test dataset, while two portions will be used for training, and the remaining two for validation. For example, if the total data size is 19,800 people, the data can be divided into five equal portions of 3,960 people.

For the training of the model, each training data from the training data sets (i.e., from two portions of the five) is used as input for a machine-learning model, along with the real sepsis situation (i.e., whether the person has sepsis or not). In other words, for the training data, it is already known whether the person has sepsis or not and so this can be used to train the machine-learning model.

To this effect, the following loss function can be used:

$$L_{classification} = \frac{1}{m}\sum_{i=1}^{m}(L_T^i + L_D^i + L_S^i) \qquad (1)$$

$$L_{T,D,S}{}^i = -y_{t,d,s}{}^i \log\left(\widehat{y_{t,d,s}}{}^i\right) - (1 - y_{t,d,s}{}^i)\log\left(1 - \widehat{y_{t,d,s}}{}^i\right) \qquad (2)$$

If the person has sepsis, the $y_{t,d,s}$ in the loss function shown in equation (2) will be 1, and if the person does not have sepsis, the $y_{t,d,s}$ will be 0.

The loss function is used to determine the loss (error) between the output of the algorithms and the given target value (i.e., $y_{t,d,s}$). The loss function can therefore be used in optimization problems with the goal of minimizing the loss. In the present training method, the loss function consists of three parts: the vital sign (time-series) loss function, $L_T$; the demographic information loss function, $L_D$; and the symptoms loss function, $L_S$.

In equation 1, $m$ represents the dataset size (i.e., how many patients are in the training data), and in equation 2, $t$, $d$ and $s$ denote the vital sign (time series) feature size, the demographic information feature size, and symptom feature size. $\widehat{y_{t,d,s}}{}^i$ indicates the probability that the patient has sepsis under the features of $t$, $d$ and $s$.

The below activation function defines the output of an artificial neural network (for example, a logistic regression model). An activation function $\sigma$ (logistic sigmoid function) will thus give the probability of sepsis as model output based on the vital sign (time-series) features, the demographic information features, and symptom features as the model input values.

$$z = W_{personal} * x_{personal} + W_{timeseries} * x_{timeseries} + W_{symptom} * x_{symptom} + bias$$

$$(3)$$

$$\hat{y} = \sigma(z) = \frac{1}{1+e^z} \qquad (4)$$

The regression model ($z$) can be taken as an example model. It should be noted that for other models, the same process applies. The output $z$ is converted into a probability of $\hat{y}$, which is input as $\widehat{y_{t,d,s}}{}^i$ in the above loss function (2). Xpersonal refers to features related to the demographic information, Xtimeseries refers to features related to vital sign values, Xsymptom refers to features related to symptom values.

For each data from the training dataset (i.e., two portions of the five), the above process will be performed. After all the data in the training dataset has undergone the above process, the same process will be applied to the data in the validation dataset (i.e., another two portions of the five). After this, the model is established with model parameters and weightings, for example.

A test dataset (i.e., the final of the five portions) can then be used to test the established model. Unlike the process in the training and validation process, the real sepsis situation is not used as additional information for the model but rather only as a test criterion.

For example, if the model output $\widehat{y_{t,d,s}}{}^i$ indicates the probability that the subject has sepsis is 75%, and this is above a warning setting threshold of 50% (or another value, for example, set by a clinician), then the indication can be taken as the patient having sepsis (i.e., being at risk of having sepsis). This indication can then be compared with the real situation to check whether the indication aligned with the real situation. In this way, the accuracy of the model can be checked.

In some embodiments of the training method, further quality check parameters can be used to check the accuracy of the model, such as recall/sensitivity (how many of the true positives are predicted to be positive), precision (positive predictive value), and accuracy.

Other commonly used model evaluation metrics are: confusion matrix (calculating the accuracy of how many predictions are correct); specificity (how many of the true negatives are predicted to be negative; ROC (receiver operating characteristic curve, plotting the true positive rate and false positive rate curves according to different thresholds); and AUC (area under curve, the area enclosed by the roc curve and the lower axis area).

The above process describes the process for establishing and testing a model based on the situation where one specific portion is used as the test dataset. However, the same thing can be done using each of the other four portions as the test dataset respectively. This means that the above process will be done five times for one model.

Thus, for one model, five model candidates will be obtained. The performance of these five model candidates can be compared to find the best model candidate from the five. Further, for different types of models (neural network, decision tree, linear model, etc.), the same process can be performed and the best model candidate is found for each type. The best models of each type of model can then be compared to one another to find the final best model for application in future (for instance, in any herein disclosed embodiment of a method for predicting a probability of sepsis in a subject).

In the final best model, if the weighting for one feature is high, then that feature is more significant for sepsis decision.

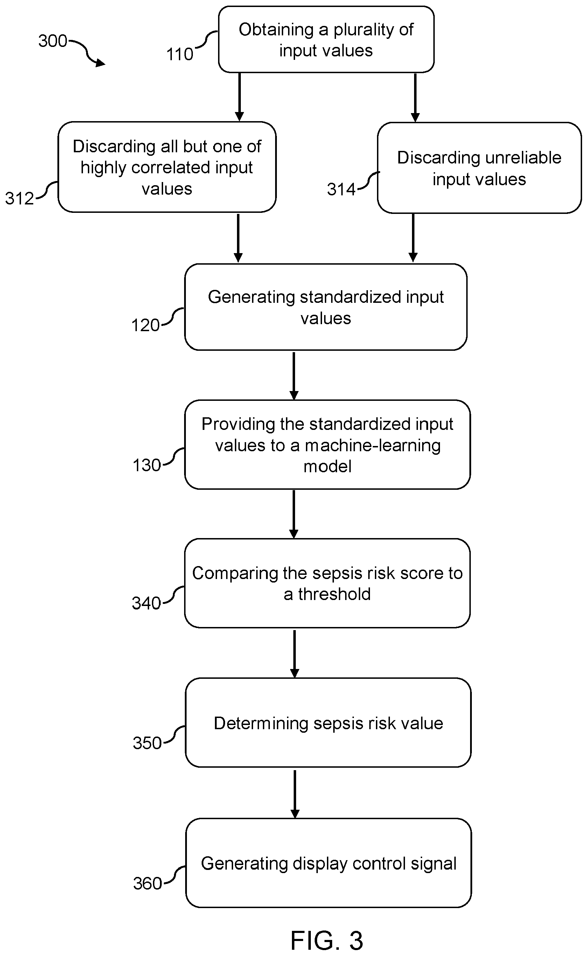

Referring now to Fig. 3, there is provided a more in-depth flow diagram of a method 300 for predicting a probability of sepsis in a subject according to a proposed embodiment. Steps 110, 120, and 130 are substantially the same as have been described in relation to method 100 of Fig. 1.

In (optional) step 312, if at least two of the input values are known to be highly correlated, then only one of the said input values is standardized and subsequently processed by the machine-learning model, i.e., all but one of the highly correlated input values will be discarded. If the input values are highly correlated, the input of more than one of them does not provide any more useful information while increasing the processing required. Discarding all but one of the values thus makes the method more efficient while maintaining its effectiveness. For example, if at least two of: systolic blood pressure; diastolic blood pressure; and mean arterial pressure are obtained, then only one of the said values is standardized and subsequently processed by the machine-learning model. These vital sign values are typically highly correlated.

In (optional) step 314, an input value is discarded if the input value is not obtained with a reliability higher than a predetermined threshold. This increases the efficiency of the method by not processing inputs which are not reliable and so unlikely to provide useful information. In other words, this feature can be understood as: if the input value is not be obtained with a sufficient reliability, then the input value is discarded and not subsequently processed. Reliability can be understood as the quality of being trustworthy for a certain input value. For example, if it is hard for a clinician for make a decision regarding the presence of one certain symptom immediately, the reliability may be relatively low.

For example, reliability can be understood as the consistent and accurate acquisition of an input value, such as heart rate, for example, over an extended period. It denotes the degree of confidence in the precision and stability of the measurement, ensuring that the recorded data faithfully represents the patient's true physiological condition. A reliable input value should therefore be capable of consistently and accurately tracking changes, for example, in heart rate, free from significant measurement errors, signal noise, or interruptions.

In step 340, the sepsis risk score is compared to a predetermined threshold score. In step 350, a sepsis risk value is determined responsive to the comparison. In this way, the sepsis risk score can be more easily interpreted. For instance, the sepsis risk score can be compared to a predetermined threshold score, and if the risk score is above the threshold, it can be determined that the subject is at risk of (having) sepsis. Conversely, if the risk score is below the threshold, it can be determined that the subject is not at risk of (having) sepsis. For example, the sepsis risk value can be one of two values indicating whether the subject is at risk of sepsis or not, respectively. In some embodiments, a user can confirm the sepsis risk value (i.e., whether the subject did have sepsis) and this diagnosis can then be fed back into the machine-learning model such that the machine-learning model can iteratively improve.

In this embodiment, the predetermined threshold score is configurable by a user. In other words, a user, such as a clinician, can adjust the threshold score to their preference. For instance, a user may find that if the threshold is set at 50%, too many subjects are being classified as being at risk of sepsis when, in reality, they are not. The user can then adjust the threshold to 70%, for example, so that only those who are determined to have a higher probability of having sepsis are labelled as being at risk of sepsis. This feature is not essential, however, and in other embodiments, the threshold may not be configurable by a user. For instance, in some embodiments, a user's confirmation of whether the subject did or did not have sepsis can be used to automatically adjust the threshold score.

In step 360, a first display control signal is generated, the first display control signal indicating the sepsis risk value. For example, this first display control signal is for controlling a display device to display an indication of the sepsis risk value. This allows a clinician to easily see whether the subject is at risk of sepsis or not.

In some embodiments, a second display control signal is generated, the second display control signal indicating the sepsis risk score. The second display control signal may be the same as the first display control signal, may replace the first display control signal, or may be separate to the first display control signal. For example, this allows a clinician to easily see the probability of the subject having sepsis.

For example, all of the disclosed features to be obtained are nearly universally available at the bedside and do not rely on laboratory tests in the ED to evaluate each algorithm and compare the predicting ability between models with or without free text integration.

For example, to minimize the unfavorable effects of differences in the scales of the input variables on the accuracy of the models that use a weighted sum of variables to fit, feature normalization or standardization plays an important role in the proposed methodology. This step means that all the numerical features (such as age, blood pressure, etc.) are standardized to a same statistic unit, for example, between 0 and 1.

For example, the pre-processing details of the input features may be as follows: (1) symptoms and signs: positive symptoms and signs are labelled as 1, and those not involved in the case or recorded as negative symptoms and signs are labelled as 0; (2) mode of arrival: ambulance is labelled as 1, walk-in is labelled as 0; (3) triage level: level 1 is labelled as 1, level 2 is labelled as 2, level III is labelled as 3, and level IV is labelled as 4 (the higher the mark is, the lower the severity is); (4) other vital sign parameters are taken directly during the process of nurse triage and standardized into the same statistical unit. Compliance with the diagnosis of sepsis is marked as 1, and non-sepsis is marked as 0. All the categorical variables are transformed into numerical values using one-hot encoding. For numerical features, they are all transformed into the same unit within [0,1] or [0,10]. For categorical features, they are transformed into numerical values, for example, as stated in points 1, 2, and 3. For example, for sex, male is labelled as 1, while female is labelled as 0. For a symptom, if the patient has it, it is labelled as 1, and if not, it is labelled as 0.

For example, in the second step, the pre-processed EMR data, vital signs, demographic data, and symptoms will be aggregated as a feature matrix. For example, each column can represent a feature (input value) and each line can represent a patient. In the third step, a multi-modal machine-learning model trained for the classification of sepsis can be used to process the input data (features) and output a prediction of a probability of sepsis in each subject.

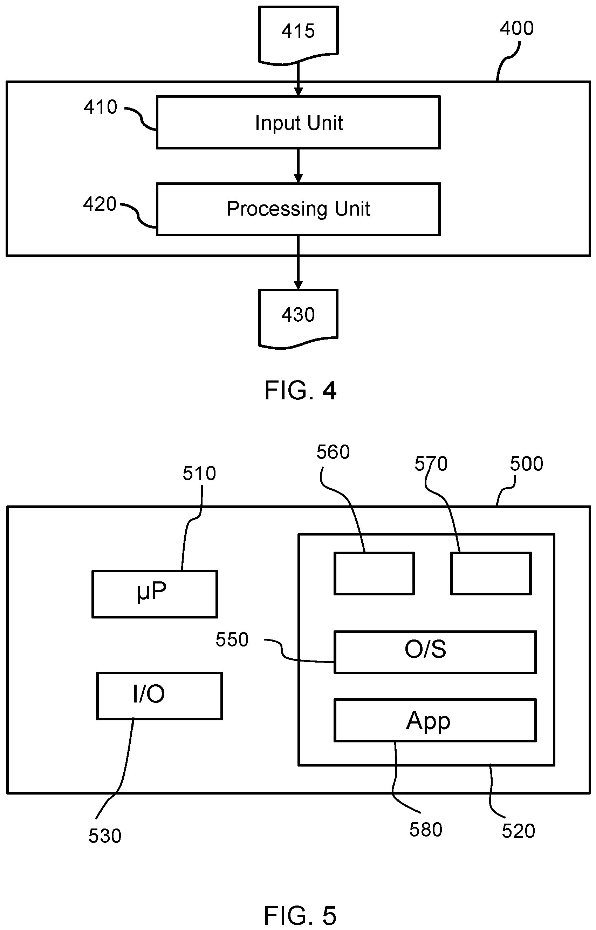

Referring now to Fig. 4, there is depicted a processing system 400 for predicting a probability of sepsis in a subject according to a proposed embodiment. The processing system 400 comprises an input unit 410 and a processing unit 420.

The processing system 400 is configured to predict a probability of sepsis in a subject by processing inputs 415. The inputs 415 comprise at least one vital sign value of the subject, at least one demographic value of the subject, and at least one symptom status value of the subject. The input unit 410 obtains the inputs 415 and the processing unit 420 is then configured to generate standardized input values by standardizing each input value to a value within a respective range or one of a set of predetermined values. The processing unit 420 is further configured to provide the standardized input values as input to a machine-learning model to generate an output 430. The output 430 comprises a sepsis risk score indicating a probability of the subject having sepsis, and the machine-learning model is trained to predict, from the standardized input data, a sepsis risk score indicating a probability of the subject having sepsis.

Fig. 5 illustrates an example of a computer 500 within which one or more parts of an embodiment may be employed. Various operations discussed above may utilize the capabilities of the computer 500. In this regard, it is to be understood that system functional blocks can run on a single computer or may be distributed over several computers and locations (e.g. connected via internet).

The computer 500 includes, but is not limited to, PCs, workstations, laptops, PDAs, palm devices, servers, storages, and the like. Generally, in terms of hardware architecture, the computer 500 may include one or more processors 510, memory 520 and one or more I/O devices 530 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 510 is a hardware device for executing software that can be stored in the memory 520. The processor 510 can be virtually any custom made or commercially available processor, a central processing unit (CPU), a digital signal processor (DSP), or an auxiliary processor among several processors associated with the computer 500, and the processor 510 may be a semiconductor based microprocessor (in the form of a microchip) or a microprocessor.

The memory 520 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 520 may incorporate electronic, magnetic, optical, and/or other types of storage media. Note that the memory 520 can have a distributed architecture, where various components are situated remote from one another, but can be accessed by the processor 510.

The software in the memory 520 may include one or more separate programs, each of which comprises an ordered listing of executable instructions for implementing logical functions. The software in the memory 520 includes a suitable operating system (O/S) 550, compiler 560, source code 570, and one or more applications 580 in accordance with exemplary embodiments. As illustrated, the application 580 comprises numerous functional components for implementing the features and operations of the exemplary embodiments. The application 580 of the computer 500 may represent various applications, computational units, logic, functional units, processes, operations, virtual entities, and/or modules in accordance with exemplary embodiments, but the application 580 is not meant to be a limitation.

The operating system 550 controls the execution of other computer programs, and provides scheduling, input-output control, file and data management, memory management, and communication control and related services. It is contemplated by the inventors that the application 580 for implementing exemplary embodiments may be applicable on all commercially available operating systems.

Application 580 may be a source program, executable program (object code), script, or any other entity comprising a set of instructions to be performed. When a source program, then the program is usually translated via a compiler (such as the compiler 560), assembler, interpreter, or the like, which may or may not be included within the memory 520, so as to operate properly in connection with the O/S 550. Furthermore, the application 580 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 530 may include input devices such as, for example but not limited to, a mouse, keyboard, scanner, microphone, camera, etc. Furthermore, the I/O devices 530 may also include output devices, for example but not limited to a printer, display, etc. Finally, the I/O devices 530 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 530 also include components for communicating over various networks, such as the Internet or intranet.

If the computer 500 is a PC, workstation, intelligent device or the like, the software in the memory 520 may further include a basic input output system (BIOS) (omitted for simplicity). The BIOS is a set of essential software routines that initialize and test hardware at start-up, start the O/S 550, and support the transfer of data among the hardware devices. The BIOS is stored in some type of read-only-memory, such as ROM, PROM, EPROM, EEPROM or the like, so that the BIOS can be executed when the computer 800 is activated.

When the computer 500 is in operation, the processor 510 is configured to execute software stored within the memory 520, to communicate data to and from the memory 520, and to generally control operations of the computer 500 pursuant to the software. The application 580 and the O/S 550 are read, in whole or in part, by the processor 510, perhaps buffered within the processor 510, and then executed.

When the application 580 is implemented in software it should be noted that the application 580 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 580 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs 1-3, and the system of Fig. 4, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 5 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

**Claims**

1. A computer-implemented method (100) for predicting a probability of sepsis in a subject, the method comprising:

    obtaining a plurality of input values (110) comprising:

        at least one vital sign value of the subject;
        at least one demographic value of the subject;
        at least one symptom status value of the subject;

    generating standardized input values (120) by standardizing each input value to a value within a respective range or one of a set of predetermined values; and
    providing the standardized input values as input to a machine-learning model (130), the machine-learning model being trained to predict, from the standardized input values, a sepsis risk score indicating a probability of the subject having sepsis.

2. The computer-implemented method of claim 1, wherein if an input value comprises a categorical value, standardizing the input value comprises setting the categorical value to one of a set of predetermined values; and/or if an input value comprises a numerical value, standardizing the input value comprises standardizing the numerical value to a value within the respective range of predetermined values.

3. The computer-implemented method of claim 2, wherein each of the at least one symptom status value is associated with a different symptom, and comprises a value indicating the presence or absence of the associated symptom.

4. The computer-implemented method of any of claims 1 to 3, wherein the machine-learning model comprises at least one of: a neural network; a decision tree; a linear model; and/or a logistic regression model.

5. The computer-implemented method of any of claims 1 to 4, wherein the method further comprises:

comparing the sepsis risk score to a predetermined threshold score (340); and
determining a sepsis risk value (350) responsive to the comparison.

6. The computer-implemented method of claim 5, wherein the predetermined threshold score is configurable by a user.

7. The computer-implemented method of claim 5 or 6, wherein the method further comprises: generating a first display control signal (360), the first display control signal indicating the sepsis risk value.

8. The computer-implemented method of any of claims 1 to 7, wherein the at least one vital sign value comprises at least one of: heart rate; body temperature; SpO2; systolic blood pressure; diastolic blood pressure; mean arterial pressure; and/or respiration rate.

9. The computer-implemented method of any of claims 1 to 8, wherein if at least two of the input values are known to be highly correlated, then only one of the said input values is standardized and subsequently processed by the machine-learning model (312).

10. The computer-implemented method of any of claims 1 to 9, further comprising discarding an input value (314) if the input value is not obtained with a reliability higher than a predetermined threshold.

11. The computer-implemented method of any of claims 1 to 10, wherein the at least one demographic value comprises at least one of: age; sex; race; mode of arrival; and/or triage level.

12. The computer-implemented method of any of claims 1 to 11, wherein the at least one symptom status value comprises at least one value indicating the presence or absence of one of the following: acutely altered mental status; abnormally frequent micturition; abnormally high body temperature; stomach ache; fever; inappetence; altered consciousness; and/or rigors.

13. The computer-implemented method of any of claims 1 to 12, wherein the method further comprises generating a second display control signal (360), the second display control signal indicating the sepsis risk score.

14. A computer program product comprising computer program code means which, when executed on a computing device having a processing system, cause the processing system to perform all of the steps of the computer-implemented method according to any of claims 1 to 13.

15. A processing system (400) for predicting a probability of sepsis in a subject, the processing system comprising:

an input unit (410) configured to obtain a plurality of input values comprising:

at least one vital sign value of the subject;
at least one demographic value of the subject; and
at least one symptom status value of the subject; and

a processing unit (420) configured to:

generate standardized input values by standardizing each input value to a value within a respective range or one of a set of predetermined values; and

provide the standardized input values as input to a machine-learning model, the machine-learning model being trained to predict, from the standardized input data, a sepsis risk score indicating a probability of the subject having sepsis.

FIG. 1

FIG. 2

300

110 — Obtaining a plurality of input values

312 — Discarding all but one of highly correlated input values

314 — Discarding unreliable input values

120 — Generating standardized input values

130 — Providing the standardized input values to a machine-learning model

340 — Comparing the sepsis risk score to a threshold

350 — Determining sepsis risk value

360 — Generating display control signal

FIG. 3

FIG. 4

FIG. 5

Europäisches
Patentamt

European
Patent Office

Office européen
des brevets

# EUROPEAN SEARCH REPORT

Application Number

**EP 23 21 6186**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/059597 A1 (CHUNG CHIEW YUAN [SG] ET AL) 4 March 2021 (2021-03-04) * the whole document, in particular figures 1, 4, 6 and 7, and paragraphs [0003] on page 1, [0048]-[0069] on pages 3-6 * | 1-15 | INV. G16H50/20 A61B5/00 G16H50/30 G16H50/50 G16H50/70 |
| A | Anonymous: "Feature scaling - Wikipedia", , 25 September 2023 (2023-09-25), XP093147565, Retrieved from the Internet: URL:https://en.wikipedia.org/w/index.php?title=Feature_scaling&oldid=1177060479 [retrieved on 2024-04-03] * the whole document * | 1-15 | |

TECHNICAL FIELDS
SEARCHED      (IPC)

G16H
A61B

The present search report has been drawn up for all claims

| Place of search | Date of completion of the search | Examiner |
|---|---|---|
| Munich | 8 April 2024 | Sasa Bastinos, Ana |

EPO FORM 1503 03.82 (P04C01)

**ANNEX TO THE EUROPEAN SEARCH REPORT
ON EUROPEAN PATENT APPLICATION NO.**

**EP 23 21 6186**

This annex lists the patent family members relating to the patent documents cited in the above-mentioned European search report.
The members are as contained in the European Patent Office EDP file on
The European Patent Office is in no way liable for these particulars which are merely given for the purpose of information.

**08-04-2024**

| Patent document cited in search report | | Publication date | Patent family member(s) | | Publication date |
|---|---|---|---|---|---|
| US 2021059597 | A1 | 04-03-2021 | CN | 112447289 A | 05-03-2021 |
| | | | EP | 3785611 A1 | 03-03-2021 |
| | | | US | 2021059597 A1 | 04-03-2021 |

EPO FORM P0459

For more details about this annex : see Official Journal of the European Patent Office, No. 12/82